# EUROPEAN PATENT SPECIFICATION

(11) **EP 3 454 806 B1**
(45) Date of publication and mention of the grant of the patent: **21.06.2023**
(21) Application number: 17795731.3
(22) Date of filing: 15.05.2017
(51) Int. Cl.: A61F 13/02, A61M 27/00, A61F 13/00

(54) **WOUND THERAPY**
WUNDTHERAPIE
TRAITEMENT DE PLAIES

(30) Priority: 13.05.2016 ZA 201603245
(43) Date of publication of application: 20.03.2019
(73) Proprietor: Eksteen, Ehren Cronje, 0182 Pretoria (ZA)
(72) Inventor: Eksteen, Ehren Cronje, 0182 Pretoria (ZA)
(74) Representative: Dummett Copp LLP
(86) International application number: PCT/IB2017/052847
(87) International publication number: WO 2017/195181

(56) References cited:
- EP-B1- 1 478 313
- WO-A1-2009/066104
- WO-A1-2009/066104
- WO-A1-2009/124100
- US-A1- 2007 055 209
- US-A1- 2014 221 907

## Description

This invention relates to wound therapy. In particular the invention relates to a wound bed access device, a negative pressure treatment device and a wound care system.

### BACKGROUND INFORMATION

There are various techniques that can be applied in treating wounds and one such technique is Negative Pressure Wound Therapy (NPWT). This technique is used to treat chronic wounds or wounds that are expected to present difficulty when healing by creating a moist environment which promotes the wound healing process.

The technique involves applying negative pressure to the wound by applying a vacuum through a sealed dressing, either continuously or intermittently. The vacuum serves to draw fluid, which may contain infectious materials, from the wound and increases blood flow to the area. The negative pressure also helps to draw the edges of the wound together. The sealed dressing is connected to a drainage tube, which removes the fluid absorbed by the dressing and transfers it to a canister.

NPWT is not without adverse effects, such as pain, bleeding, infection, other complications. Proper monitoring of the patient and wound, as well as regular changes of the dressing, can help to decrease the risk of any adverse effects.

The inventor is aware of the technique and the possible adverse effects associated with it and aims to provide an invention which will improve the current use of NPWT.

### SUMMARY OF THE INVENTION

Broadly according to a first aspect of the invention there is provided a wound bed access device for providing access to a wound bed from outside a closed wound, which includes
a self-sealing valve connector defining a flow path which is ordinarily closed, wherein the self-sealing valve connector includes a movable sealing member movable within the self-sealing valve connector and that is opened when a complementary opening connector of a supply or extraction apparatus, is connected to the self-sealing valve connector and allows flow in and out; and
a wound dressing interface, shaped and dimensioned to be enclosed within a wound dressing covering a wound, the wound dressing interface being in the form of a flexible pad and having an underside which in use faces the wound bed and a top side having a planar surface for the wound dressing to adhere over, the self-sealing valve connector being connected to the top side of the wound dressing interface, with the flow path passing from the self-sealing valve connector through the wound dressing interface, in use the self-sealing valve connector is positioned outside the wound dressing thereby providing access to the wound bed from outside the closed wound, and allowing a supply or extraction apparatus to be intermittently connected or disconnected from the self-sealing valve connector.

In one embodiment, the self-sealing valve connector may be distal from the wound dressing interface, the self-sealing valve connector and wound dressing interface having a distal access tube extending between them.

The distal access tube may be detachable from any one or both of the self-sealing valve connector and wound dressing interface. Alternatively, the wound bed access device may be of unitary construction.

The flexible pad may be planar and extend perpendicularly to the flow path through the self-sealing valve connector. As the self-sealing valve connector is connected to the top side of the wound dressing interface, in use the wound dressing interface can be sealed within the wound dressing covering the wound, such that the self-sealing valve connector is outside the sealed wound.

When the complementary opening connector is disconnected from the self-sealing valve connector, the valve connector returns to a closed position, preventing flow there through.

The self-sealing valve connector may be in the form of a quick-disconnect coupler.

The self-sealing valve connector may be in the form of a luer type connector.

In one embodiment the self-sealing valve connector may be in the form of a female luer access connector. In such an embodiment the opening connector may be in the form of male luer taper. In another embodiment the self-sealing valve connector may be in the form of a male luer connector and the opening connector may be in the form of a female luer connector.

The self-sealing valve connector may include a movable sealing member that controls the flow of fluid and/or gas through the self-sealing valve connector.

The movable sealing member may be longitudinally movable within the valve connector. The self-sealing valve connector may include a biasing member such as a spring for keeping the sealing member in an ordinarily closed position. When the opening connector is connected to the self-sealing valve connector the movable sealing member is pushed down providing a flow path through the self-sealing valve connector.

The movable sealing member may be in the form of any one of a ball, a sphere, a diaphragm, a flap or the like.

In one example the movable sealing member may be in the form of a hinged flap.

The hinged flap may be movable within the valve connector. The hinged flap is ordinarily in a closed position, in which the flap closes the flow path, and in which the flap extends transversely to the flow path. When the opening connector is connected to the self-sealing valve connector the flap is pushed down providing a flow path through the self-sealing valve connector.

In another embodiment the self-sealing valve connector may include a septum having a longitudinal slit. The opening connector may push through the slit when connected to the self-sealing valve connector, providing a flow path. When the opening connector is disconnected from the self-sealing valve connector, the slit in the septum closes and the flow path is closed off.

The wound bed access device may further include a wound bed extension tube, extending from the wound dressing interface on an opposed side to the self-sealing valve connector, being in flow communication with the flow path, in use to connect the self-sealing valve connector with the wound bed.

The wound bed extension tube may include a plurality of apertures, longitudinal grooves or the like, spaced along the length of the extension tube, through which fluid and/or gas may move. In use, when fluid and/or gas is introduced through the self-sealing valve connector, the fluid and/or gas is distributed to the wound bed through the plurality of apertures. Alternatively, when fluid and/or wound debris is extracted from the self-sealing valve connector, the fluid and/or wound debris is drawn in through the plurality of apertures.

In one embodiment, the wound bed extension tube may be connectable to the distal access tube at the wound dressing interface, such that in use the flow path extends through the self-sealing valve connector, distal access tube, wound dressing interface and wound bed extension tube into the wound. Alternatively, the distal access tube and wound bed extension tube may be of a single tube passing through the wound dressing interface, a portion of the tube extending operatively above the wound dressing interface defining the distal access tube and the portion operatively below the wound dressing interface defining the wound bed extension tube.

In accordance with a second aspect of the invention, there is provided a negative pressure treatment device, which includes
a wound bed access device according to the first aspect of the invention; and
a reduced pressure port being integrated with the wound dressing interface, the reduced pressure port being connectable to a reduced pressure source, the negative pressure treatment device is operable to provide negative pressure to the wound.

The reduced pressure port may be connectable to a reduced pressure source in the form of a vacuum pump.

The negative pressure treatment device may be operable to provide negative pressure and fluid and/or gas supply to the wound.

According to a further aspect of the invention, there is provided a wound care system, which includes
a wound bed access device as described, positioned proximate a wound; and
an airtight wound dressing, in use adhering around the self-sealing valve connector over the wound dressing interface, to seal the wound bed access device onto the wound.

The airtight wound dressing may be in the form of an adhesive film, surgical drape or the like. The airtight wound dressing may be adhered to intact skin peripheral to the wound and to the wound dressing interface of the wound bed access device, to provide an airtight seal around the wound.

In one embodiment, the wound care system may further include a reduced pressure port, sealed through the airtight seal around the wound. The reduced pressure port may be connected to the airtight wound dressing to provide flow communication to the sealed space created between the wound and the wound dressing. The reduced pressure port may be connectable to a reduced pressure source.

The airtight seal may be a planar sheet with two apertures for receiving the self-sealing valve connector and reduced pressure port there through, and sealing them over the wound.

In another embodiment, the wound bed access device is integrated into a negative pressure treatment device as described, which includes the wound bed access device, a wound bed extension tube and a reduced pressure port which is integrated with the wound dressing interface, the negative pressure treatment device being operable to be sealed over the wound. The wound dressing interface may be in the form of a flexible pad.

The wound care system may further include an absorbent insert. The absorbent insert may be in the form of any of: foam, gauze or the like. In a preferred embodiment the absorbent insert may be in the form of a foam pad. The foam pad may be of open cell polyurethane, polyether foam or the like. The adsorbent insert may be shaped to match the shape of the wound cavity. In use, the wound dressing interface is placed on the absorbent insert and the airtight wound dressing is sealed there over.

The wound care system may further include one or more supply apparatuses, for supplying gas and/or liquid to the wound bed. The supply apparatus may be connectable to the self-sealing valve connector, via an opening connector. The supply apparatus may be in the form of a fluid drip line, a gas line, a syringe or the like. The supply apparatus may include a pump, for forcing the gas and/or liquid through the self-sealing valve connector. The fluid drip line may be operable to administer medication or the like to the wound site. Examples of the medication may be in the form of any one or more of: antibiotics, pain relief medication, anti-inflammatories, or the like. The gas line may be operable to administer gas to the wound site. Examples of the gas may be in the form of a medicinal gas, O2, O3 or the like. The supply apparatus may intermittently be connected or disconnected from the self-sealing valve connector.

The wound care system may also include an extraction apparatus connectable to the self-sealing valve connector, for extracting wound fluid and/or debris from the wound bed. The extraction apparatus may be in the form of a syringe. The extraction apparatus may intermittently be connected or disconnected from the self-sealing valve connector.

The wound care system may include a receptacle connectable to the extraction apparatus, alternatively to the reduced pressure port for collecting wound fluid and/or debris which has been extracted from the wound bed. The receptacle may be disconnected from the extraction apparatus to dispose of the wound fluid and/or wound debris before being replaced. Alternatively, the receptacle may be in the form of a disposable receptacle.

The wound care system may include a reduced pressure source. The reduced pressure source may be in the form of a vacuum pump connected to the reduced pressure port for providing negative pressure at the wound site.

The vacuum pump may fluctuate the pressure, such that the absorbent insert switches between soaking and emptying. In another example the vacuum pump may draw a vacuum continuously.

The invention is now described, by way of non-limiting example, with reference to the accompanying drawings:

### DRAWING(S)

In the drawings:
Figure 1 shows a three dimensional view of a wound bed access device in accordance with a first aspect of the invention according to one embodiment;
Figure 2 shows shows a sectional view of a wound bed access device in accordance with a first aspect of the invention according to another embodiment;
Figure 3 shows a sectional view of a wound care system in use;
Figure 4 shows a sectional view of a negative pressure treatment device in accordance with a further aspect of the invention;
Figure 5 shows another example of a wound care system.

In the drawings, like reference numerals denote like parts of the invention unless otherwise indicated.

### EMBODIMENT OF THE INVENTION

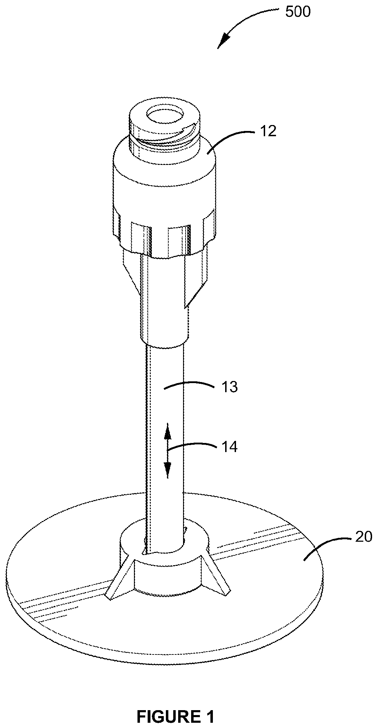

In Figure 1 reference numeral (500) refers to a wound bed access device providing access to a wound bed from outside a sealed wound. The wound bed access device (500) includes a self-sealing valve connector (12) defining a flow path (14) which is ordinarily closed and is opened when a complementary opening connector is connected to the self-sealing valve connector (12). The wound bed access device (500) also includes a wound dressing interface (20) which is distal to the self-sealing valve connector, the self-sealing valve connector (12) and the wound dressing interface (20) having a distal access tube (13) extending between them, with the flow path (14) passing from the self-sealing valve connector (12) through the wound dressing interface (20).

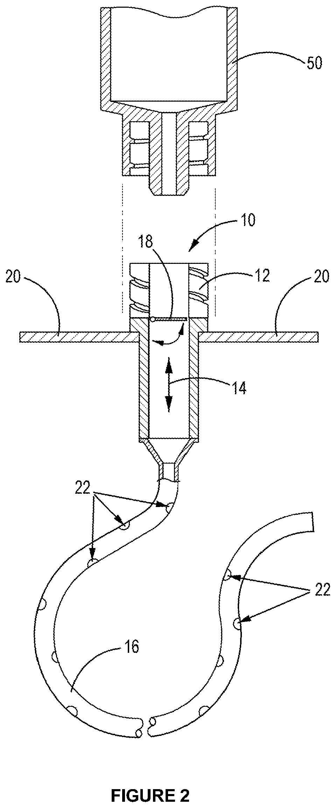

In Figure 2 reference numeral (10) refers to another embodiment of the wound bed access device (10). The self-sealing valve connector (12) defining a flow path (14) that is ordinarily closed and is opened when a complementary opening connector (50) is connected to the self-sealing valve connector (12). In this embodiment, the self-sealing valve connector (12) has a wound bed extension tube (16) extending from it and is in flow communication with the flow path (14), in use to connect the self-sealing valve connector (12) with the wound bed.

As shown in Figure 2, the self-sealing valve connector (12) is ordinarily in a closed position. The self-sealing valve connector (12) is connectable to a complementary opening connector (50), which upon connection with the self-sealing valve connector (12), opens the self-sealing valve connector (12) to allow flow through the valve. When the complementary opening connector (50) is disconnected from the self-sealing valve connector (12), the valve connector (12) returns to a closed position, preventing flow there through.

The self-sealing valve connector (12) includes a movable sealing member (18) that controls the flow of fluid and/or gas through the self-sealing valve connector (12). In this example the movable sealing member (18) is in the form of a hinged flap (18).

The hinged flap (18) is movable within the self-sealing valve connector (12). The hinged flap (18) is ordinarily in a closed position, in which the flap (18) closes the flow path (14), and in which the flap extends transversely to the flow path (14). When the opening connector (50) is connected to the self-sealing valve connector (12) the flap (18) is pushed down providing a flow path (14) through the self-sealing valve connector (12).

In this example, the wound dressing interface (20) is in the form of a flexible pad into which the self-sealing valve connector (12) is integrated. The flexible pad (20) provides a planar surface for a wound cover to adhere over, the flexible pad (20) being planar and extending perpendicularly to the flow path through the self-sealing valve connector (12). In this example the flexible pad (20) is of silicone, is about 3 mm thick and approximately 6 cm wide.

The wound bed extension tube (16) includes a plurality of apertures (22) spaced along the length of the extension tube (16), through which fluid and/or gas can move. In use, when fluid and/or gas is introduced through the self-sealing valve connector (12), the fluid and/or gas is distributed to the wound bed through the plurality of apertures (22). Alternatively, when fluid and/or wound debris is extracted from the self-sealing valve connector (12), the fluid and/or wound debris is drawn in through the plurality of apertures (22).

In this example the wound bed extension tube (16) is of Polyvinyl Chloride (PVC) tubing. The wound bed extension tube (16) is about 20 cm long and includes ten apertures (22) spaced 1cm apart.

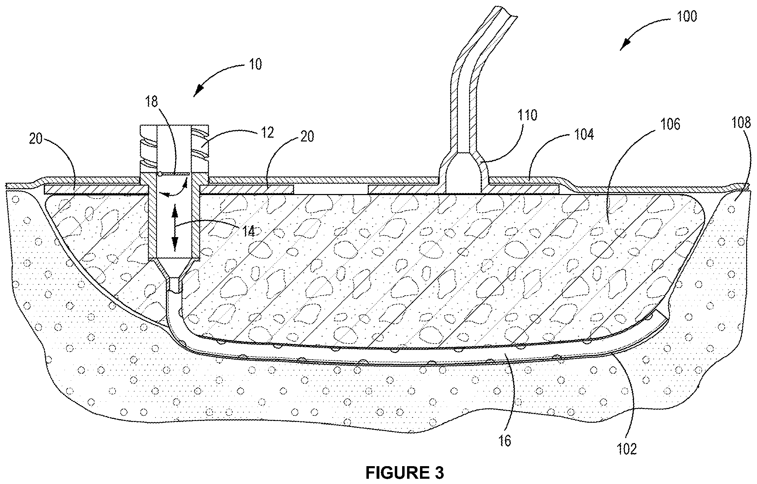

As shown in Figure 3, according to a further aspect of the invention there is provided a wound care system (100). The wound care system (100) includes a wound bed access device (10) as described, positioned proximate a wound, with a wound bed extension tube (16) positioned in the wound bed (102) of the wound and an airtight wound dressing (104) adhering around the self-sealing valve connector (12) over the wound dressing interface (20), to seal the wound bed access device (10) onto the wound.

The wound care system (100) further includes an absorbent insert (106). In this example the absorbent insert (106) is in the form of a foam pad. The foam pad (106) is of open cell polyurethane. The foam pad (106) is shaped to match the shape of the wound cavity.

In this example the airtight wound dressing (104) is in the form of an adhesive film. In use, the airtight wound dressing (104) is adhered to intact skin (108) peripheral the wound site and to the flexible pad (20) of the wound bed access device (10), to provide an airtight seal around the wound.

In this example the wound care system (100) further includes a reduced pressure port (110), sealed through the airtight seal around the wound. The reduced pressure port (110) is connected to the airtight wound dressing (104) to provide flow communication to the sealed space created between the wound and the wound dressing (104).

The reduced pressure port (110) is connectable to a reduced pressure source, such as a vacuum pump.

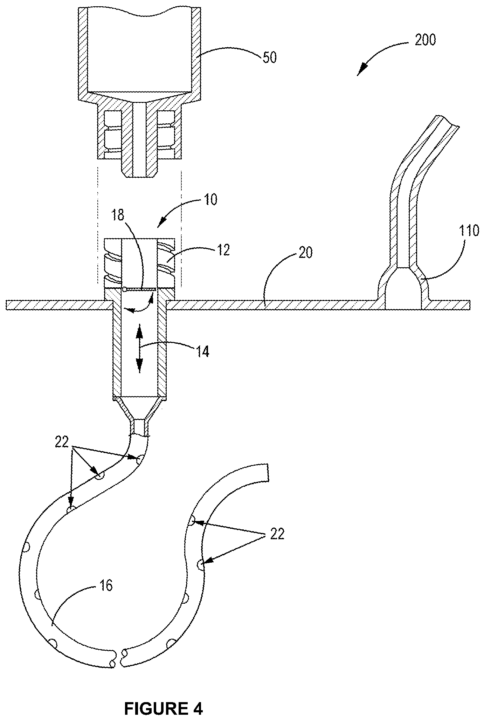

Figure 4 shows a negative pressure treatment device (200), which includes a wound bed access device (10) as described, a wound bed extension tube (16) and a reduced pressure port (110) which is integrated into the flexible pad (20), the negative pressure treatment device (200) being operable to be sealed over a wound.

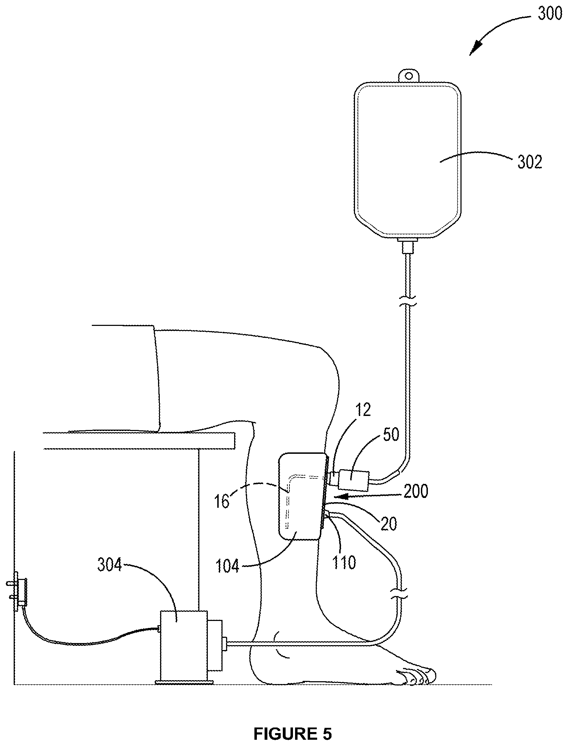

Figure 5 shows a wound care system (300), which includes a wound bed access device (10) as described positioned on a wound with a wound bed extension tube (16) positioned in the wound bed of the wound and an airtight wound dressing (104) adhering around the self-sealing valve connector (12), to seal the wound bed access device (10) onto the wound.

In this example the wound bed access device (10) is integrated into the negative pressure treatment device (200) as shown in Figure 4, which includes the wound bed access device (10), a wound bed extension tube (16) and a reduced pressure port (110) which is integrated into the flexible pad (20), the negative pressure treatment device (200) is sealed over the wound.

The wound care system (300) includes an absorbent insert (not shown). The absorbent insert is in the form of a foam pad of open cell polyurethane, which is shaped to match the shape of the wound cavity.

The airtight wound dressing (104) is in the form of an adhesive film which is adhered to intact skin peripheral the wound site and to the flexible pad (20) of the negative pressure treatment device (200).

The wound care system (300) further includes supply apparatuses, for supplying gas and/or liquid to the wound bed. The supply apparatus is connectable to the self-sealing valve connector (12), via an opening connector (50). In this example the supply apparatus is in the form of a fluid drip line (302), but the fluid drip line (302) may be disconnected from the self-sealing valve connector (12) and switched with a gas line, syringe or the like.

The fluid drip line (302) is operable to administer medication or the like to the wound site. Examples of the medication may be in the form of any one or more of: antibiotics, pain relief medication, anti-inflammatories or the like.

The wound care system (300) also includes an extraction apparatus (not shown) connectable to the self-sealing valve connector (12), for extracting wound fluid and/or debris from the wound bed. In use, the extraction device is operable to extract samples from the wound, such that the microbial load or the like can be tested at time intervals without opening the wound.

The wound care system (300) also includes a reduced pressure source (304). In this example the reduced pressure source (304) is in the form of a vacuum pump having a storage canister connected to the reduced pressure port (110) for providing negative pressure at the wound site.

In this example the vacuum pump (304) fluctuates the pressure, such that the absorbent insert (not shown) switches between soaking and emptying.

The inventor believes that the invention provides a new negative pressure treatment device being operable to provide negative pressure, fluid and/or gas supply to the wound site and access to the wound for extracting samples.

## Claims

1. A wound bed access device (500, 10) for providing access to a wound bed from outside a closed wound, which includes
a self-sealing valve connector (12) defining a flow path (14) which is ordinarily closed, wherein the self-sealing valve connector (12) includes a movable sealing member (18) movable within the self-sealing valve connector (12) and that is opened when a complementary opening connector (50) of a supply or extraction apparatus, is connected to the self-sealing valve connector (12) and allows flow in and out; and
a wound dressing interface (20), shaped and dimensioned to be enclosed within a wound dressing covering a wound, the wound dressing interface (20) being in the form of a flexible pad and having an underside which in use faces the wound bed and a top side having a planar surface for the wound dressing to adhere over, the self-sealing valve connector (12) being connected to the top side of the wound dressing interface (20), with the flow path (14) passing from the self-sealing valve connector (12) through the wound dressing interface (20), in use the self-sealing valve connector (12) is positioned outside the wound dressing thereby providing access to the wound bed from outside the closed wound, and allowing a supply or extraction apparatus to be intermittently connected or disconnected from the self-sealing valve connector (12).

2. The wound bed access device as claimed in claim 1, in which the self-sealing valve connector (12) is integrated on a top side of the wound dressing interface (20).

3. The wound bed access device as claimed in claim 1, in which the self-sealing valve connector (12) is distal from the wound dressing interface (20) and which includes a distal access tube (13) which extends between the self-sealing valve connector (12) and wound dressing interface (20).

4. The wound bed access device as claimed in claim 1, in which the wound dressing interface (20) in the form of a flexible pad provides a planar surface for a wound cover to adhere over, the flexible pad being planar and extending perpendicular to the flow path through the self-sealing valve connector (12).

5. The wound bed access device as claimed in claim 1, in which the self-sealing valve connector (12) is in the form of a quick-disconnect coupler.

6. The wound bed access device as claimed in claim 1, in which the self-sealing valve connector (12) is in the form of a luer type connector.

7. The wound bed access device as claimed in claim 1, which includes a wound bed extension tube (16) to be positioned in the wound bed, the wound bed extension tube (16) extending from the self-sealing valve connector (12) and in flow communication with the flow path (14), in use to connect the self-sealing valve connector (12) with the wound bed.

8. The wound bed access device as claimed in claim 7, in which the wound bed extension tube (16) includes a plurality of apertures (22) spaced along the length of the extension tube (16), through which fluid and/or gas move, in use fluid and/or gas is introduced through the self-sealing valve connector (12), the fluid and/or gas being distributed to the wound bed through the plurality of apertures (22), or fluid and/or wound debris is extracted from the self-sealing valve connector (12), the fluid and/or wound debris being drawn in through the plurality of apertures (22).

9. A negative pressure treatment device (200), which includes
a wound bed access device (500, 10) as claimed in claim 1; and
a reduced pressure port (110) being integrated with the wound dressing interface (20), the reduced pressure port (110) being connectable to a reduced pressure source, the negative pressure treatment device is operable to provide negative pressure to the wound.

10. A wound care system (300), which includes
a wound bed access device (500, 10) as claimed in claim 1, positioned proximate a wound; and
an airtight wound dressing (104), in use adhering around the self-sealing valve connector (12) over the wound dressing interface (20), to seal the wound bed access device (500, 10) onto the wound.

11. The wound care system as claimed in claim 10, in which the wound care system (300) includes an absorbent insert shaped to match the shape of a wound cavity.

12. The wound care system as claimed in claim 10 or claim 11, in which the wound care system (300) includes one or more supply apparatuses and/or an extraction apparatus, for supplying gas and/or liquid to a wound bed or for extracting wound fluid and/or debris from the wound bed respectively, the one or more supply apparatuses and/or extraction apparatus are connectable to the self-sealing valve connector (12), via the opening connector (50).

13. The wound care system as claimed in claim 10, which includes a separate reduced pressure port (110) sealable through the airtight wound dressing (104) around the wound, the reduced pressure port (110) is connected through the airtight wound dressing (104) to provide flow communication to the sealed space created between the wound and the wound dressing (104), and in which the wound care system (300) includes a reduced pressure source (304) in the form of a vacuum pump connectable to the reduced pressure port (110) for providing negative pressure at the wound.

14. The wound care system as claimed in claim 10, in which the wound bed access device (10) includes a reduced pressure port (110) integrated with the wound dressing interface (20), such that the wound dressing interface (20) defines two flow paths there through, a first associated with the self-sealing valve connector (12) and a second associated with the reduced pressured port (110) and in which the wound care system (300) includes a reduced pressure source (304) in the form of a vacuum pump connected to the reduced pressure port (110) for providing negative pressure at the wound.

## Patentansprüche

1. Wundbettzugangsvorrichtung (500, 10) zur Bereitstellung von Zugang zu einem Wundbett von außerhalb einer geschlossenen Wunde, die aufweist:
einen selbstabdichtenden Ventilanschluss (12), der einen Strömungsweg (14) definiert, der gewöhnlich geschlossen ist, wobei der selbstabdichtende Ventilanschluss (12) ein bewegliches Dichtelement (18) aufweist, das innerhalb des selbstabdichtenden Ventilanschlusses (12) beweglich ist und das geöffnet wird, wenn ein komplementärer Öffnungsanschluss (50) eines Zufuhr- oder Extraktionsgeräts mit dem selbstabdichtenden Ventilanschluss (12) verbunden wird, und Ein- und Ausströmen erlaubt; und
eine Wundverbandschnittstelle (20), die geformt und bemessen ist, in einen Wundverband, der eine Wunde bedeckt, eingeschlossen zu werden, wobei die Wundverbandschnittstelle (20) die Form eines flexiblen Kissens aufweist und eine Unterseite, die in Verwendung zu dem Wundbett zeigt, und eine Oberseite mit einer ebenen Oberfläche zum Haften des Wundverbands darüber aufweist, wobei der selbstabdichtende Ventilanschluss (12) mit der Oberseite der Wundverbandschnittstelle (20) verbunden ist, wobei der Strömungsweg (14) von dem selbstabdichtenden Ventilanschluss (12) durch die Wundverbandschnittstelle (20) verläuft, wobei in Verwendung der selbstabdichtende Ventilanschluss (12) außerhalb des Wundverbands angeordnet ist, um Zugang zu dem Wundbett von außerhalb der geschlossenen Wunde bereitzustellen und zu erlauben, dass ein Zufuhr- oder Extraktionsgerät intermittierend mit dem selbstabdichtenden Ventilanschluss (12) verbunden oder davon getrennt werden kann.

2. Wundbettzugangsvorrichtung gemäß Anspruch 1, wobei der selbstabdichtende Ventilanschluss (12) an einer Oberseite der Wundverbandschnittstelle (20) integriert ist.

3. Wundbettzugangsvorrichtung gemäß Anspruch 1, wobei der selbstabdichtende Ventilanschluss (12) distal von der Wundverbandschnittstelle (20) angeordnet ist, und die ein distales Zugangsrohr (13) aufweist, das zwischen dem selbstabdichtenden Ventilanschluss (12) und der Wundverbandschnittstelle (20) verläuft.

4. Wundbettzugangsvorrichtung gemäß Anspruch 1, wobei die Wundverbandschnittstelle (20) in der Form eines flexiblen Kissens eine ebene Oberfläche zum Haften einer Wundabdeckung darüber bereitstellt, wobei das flexible Kissen eben ist und senkrecht zu dem Strömungsweg durch den selbstabdichtenden Ventilanschluss (12) verläuft.

5. Wundbettzugangsvorrichtung gemäß Anspruch 1, wobei der selbstabdichtende Ventilanschluss (12) in der Form eines schnell lösbaren Kopplungselements vorliegt.

6. Wundbettzugangsvorrichtung gemäß Anspruch 1, wobei der selbstabdichtende Ventilanschluss (12) in der Form eines Verbindungselements vom Luer-Typ vorliegt.

7. Wundbettzugangsvorrichtung gemäß Anspruch 1, das einen Wundbett-Verlängerungsschlauch (16) zur Positionierung in dem Wundbett aufweist, wobei der Wundbett-Verlängerungsschlauch (16), der sich von dem selbstabdichtenden Ventilanschluss (12) erstreckt und in Strömungsverbindung mit dem Strömungsweg (14) steht, in Verwendung zum Verbinden des selbstabdichtenden Ventilanschlusses (12) mit dem Wundbett dient.

8. Wundbettzugangsvorrichtung gemäß Anspruch 7, wobei der Wundbett-Verlängerungsschlauch (16) eine Vielzahl von entlang der Länge des Verlängerungsschlauchs (16) beabstandeten Öffnungen (22) aufweist, durch die sich Fluid und/oder Gas bewegt, wobei in Verwendung Fluid und/oder Gas durch den selbstabdichtenden Ventilanschluss (12) eingeführt wird, das Fluid und/oder Gas durch die Vielzahl von Öffnungen (22) an das Wundbett ausgegeben wird, oder Fluid und/oder Wundrückstände aus dem selbstabdichtenden Ventilanschluss (12) extrahiert wird, wobei das Fluid und/oder die Wundrückstände durch die Vielzahl von Öffnungen (22) gezogen werden.

9. Unterdruckbehandlungsvorrichtung (200), die aufweist:
eine Wundbettzugangsvorrichtung (500, 10) gemäß Anspruch 1; und
einen Unterdruckanschluss (110), der in der Wundverbandschnittstelle (20) integriert ist, wobei der Unterdruckanschluss (110) mit einer Unterdruckquelle verbindbar ist, wobei die Unterdruckbehandlungsvorrichtung derart betrieben werden kann, dass sie Unterdruck auf die Wunde ausübt.

10. Wundpflegesystem (300), das umfasst:
eine Wundbettzugangsvorrichtung (500, 10) gemäß Anspruch 1, die nahe einer Wunde angeordnet ist; und
einen luftdichten Wundverband (104), der in Verwendung um den selbstabdichtenden Ventilanschluss (12) über der Wundverbandschnittstelle (20) haftet, um die Wundbettzugangsvorrichtung (500, 10) an der Wunde abzudichten.

11. Wundpflegesystem gemäß Anspruch 10, wobei das Wundpflegesystem (300) einen Absorptionsmitteleinsatz aufweist, der geformt ist, der Form einer Wundhöhle zu entsprechen.

12. Wundpflegesystem gemäß Anspruch 10 oder Anspruch 11, wobei das Wundpflegesystem (300) ein oder mehrere Zufuhrgeräte und/oder ein Extraktionsgerät aufweist, um einem Wundbett Gas und/oder Flüssigkeit zuzuführen bzw. um Wundfluid und/oder Rückstände aus dem Wundbett zu extrahieren, wobei das eine oder die mehreren Zufuhrgeräte und/oder das Extraktionsgerät über den Öffnungsanschluss (50) mit dem selbstabdichtenden Ventilanschluss (12) verbindbar sind.

13. Wundpflegesystem gemäß Anspruch 10, das einen getrennten Unterdruckanschluss (110) aufweist, der durch den luftdichten Wundverband (104) um die Wunde abdichtbar ist, wobei der Unterdruckanschluss (110) durch den luftdichten Wundverband (104) verbunden ist, um Strömungskommunikation zu dem abgedichteten Raum, der zwischen der Wunde und dem Wundverband (104) gebildet ist, bereitzustellen, und wobei das Wundpflegesystem (300) eine Unterdruckquelle (304) in der Form einer Unterdruckpumpe aufweist, die mit dem Unterdruckanschluss (110) verbindbar ist, um Unterdruck an der Wunde bereitzustellen.

14. Wundpflegesystem gemäß Anspruch 10, wobei die Wundbettzugangsvorrichtung (10) einen Unterdruckanschluss (110) aufweist, der in der Wundverbandschnittstelle (20) integriert ist, so dass die Wundverbandschnittstelle (20) zwei hindurchgehende Strömungswege definiert, einen ersten, der mit dem selbstabdichtenden Ventilanschluss (12) verbunden ist, und einen zweiten, der mit dem Unterdruckanschluss (110) verbunden ist, und wobei das Wundpflegesystem (300) eine Unterdruckquelle (304) in der Form einer Unterdruckpumpe aufweist, die mit dem Unterdruckanschluss (110) verbunden ist, um Unterdruck an der Wunde bereitzustellen.

## Revendications

1. Dispositif d'accès au lit de la plaie (500, 10) pour fournir un accès au lit de la plaie depuis l'extérieur d'une plaie fermée, qui inclut
un connecteur à valve auto-scellant (12) définissant un chemin d'écoulement (14) qui est ordinairement fermé, dans lequel le connecteur à valve auto-scellant (12) inclut un élément scellant mobile (18) mobile à l'intérieur du connecteur à valve auto-scellant (12) et qui est ouvert lorsqu'un connecteur d'ouverture complémentaire (50) d'un appareil de fourniture ou d'extraction, est connecté au connecteur à valve auto-scellant (12) et permet l'écoulement entrant et sortant; et
une interface de pansement (20), formée et dimensionnée pour être incluse à l'intérieur d'un pansement recouvrant une plaie, l'interface de pansement (20) étant sous la forme d'une plaque flexible et ayant un côté inférieur qui, lors de l'utilisation, fait face au lit de la plaie et un côté supérieur ayant une surface plane pour que le pansement y adhère, le connecteur à valve auto-scellant (12) étant connecté au côté supérieur de l'interface de pansement (20), le chemin d'écoulement (14) passant du connecteur à valve auto-scellant (12) à travers l'interface de pansement (20), lors de l'utilisation le connecteur à valve auto-scellant (12) est positionné à l'extérieur du pansement fournissant ainsi un accès au lit de la plaie depuis l'extérieur de la plaie fermée, et permettant à un appareil de fourniture ou d'extraction d'être connecté ou déconnecté du connecteur à valve auto-scellant (12) de manière intermittente.

2. Dispositif d'accès au lit de la plaie selon la revendication 1, dans lequel le connecteur à valve auto-scellant (12) est intégré sur un côté supérieur de l'interface de pansement (20).

3. Dispositif d'accès au lit de la plaie selon la revendication 1, dans lequel le connecteur à valve auto-scellant (12) est distal par rapport à l'interface de pansement (20) et qui inclut un tube d'accès distal (13) qui s'étend entre le connecteur à valve auto-scellant (12) et l'interface de pansement (20).

4. Dispositif d'accès au lit de la plaie selon la revendication 1, dans lequel l'interface de pansement (20) sous la forme d'une plaque flexible fournit une surface plane pour qu'une couverture de plaie adhère par-dessus, la plaque flexible étant plane et s'étendant perpendiculairement au chemin d'écoulement à travers le connecteur à valve auto-scellant (12).

5. Dispositif d'accès au lit de la plaie selon la revendication 1, dans lequel le connecteur à valve auto-scellant (12) est sous la forme d'un coupleur à déconnexion rapide.

6. Dispositif d'accès au lit de la plaie selon la revendication 1, dans lequel le connecteur à valve auto-scellant (12) est sous la forme d'un connecteur de type luer.

7. Dispositif d'accès au lit de la plaie selon la revendication 1, qui inclut un tube d'extension de lit de la plaie (16) destiné à être positionné dans le lit de la plaie, le tube d'extension de lit de la plaie (16) s'étendant depuis le connecteur à valve auto-scellant (12) et en communication d'écoulement avec le chemin d'écoulement (14), lors de l'utilisation pour connecter le connecteur à valve auto-scellant (12) avec le lit de la plaie.

8. Dispositif d'accès au lit de la plaie selon la revendication 7, dans lequel le tube d'extension de lit de la plaie (16) inclut une pluralité d'ouvertures (22) espacées sur la longueur du tube d'extension (16), à travers lesquelles du fluide et/ou du gaz se déplacent, lors de l'utilisation du fluide et/ou du gaz est introduit à travers le connecteur à valve auto-scellant (12), le fluide et/ou le gaz étant distribué jusqu'au lit de la plaie à travers la pluralité d'ouvertures (22), ou le fluide et/les débris de la plaie sont extraits par le connecteur à valve auto-scellant (12), le fluide et/ou les débris de la plaie étant aspirés à travers la pluralité d'ouvertures (22).

9. Dispositif de traitement à pression négative (200), qui inclut
un dispositif d'accès au lit de la plaie (500, 10) selon la revendication 1; et
un orifice à pression réduite (110) intégré à l'interface de pansement (20), l'orifice à pression réduite (110) pouvant être connecté à une source de pression réduite, le dispositif de traitement à pression négative peut être actionné pour fournir une pression négative à la plaie.

10. Système de soin des plaies (300), qui inclut
un dispositif d'accès au lit de la plaie (500, 10) selon la revendication 1, positionné à proximité d'une plaie, et
un pansement étanche à l'air (104), lors de utilisation adhérant autour du connecteur à valve auto-scellant (12) au-dessus de l'interface de pansement (20), pour sceller le dispositif d'accès au lit de la plaie (500, 10) sur la plaie.

11. Système de soin des plaies selon la revendication 10, dans lequel le système de soin des plaies (300) inclut un insert absorbant formé pour correspondre à la forme d'une cavité de plaie.

12. Système de soin des plaies selon la revendication 10 ou la revendication 11, dans lequel le système de soin des plaies (300) inclut un ou plusieurs appareils de fourniture et/ou un appareil d'extraction, pour la fourniture de gaz et/ou de liquide au lit de la plaie ou pour extraire du liquide de la plaie et/ou des débris du lit de la plaie respectivement, les un ou plusieurs appareils de fourniture et/ou l'appareil d'extraction peuvent être connectés au connecteur à valve auto-scellant (12), via le connecteur d'ouverture (50).

13. Système de soin des plaies selon la revendication 10, qui inclut un orifice à pression réduite séparé (110) pouvant être scellé à travers le pansement étanche à l'air (104) autour de la plaie, l'orifice à pression réduite (110) est connecté à travers le pansement étanche à l'air (104) pour fournir une communication d'écoulement jusqu'à l'espace scellé créé entre la plaie et le pansement (104), et dans lequel le système de soin des plaies (300) inclut une source de pression réduite (304) sous la forme d'une pompe à vide pouvant être connectée à l'orifice à pression réduite (110) pour fournir une pression négative au niveau de la plaie.

14. Système de soin des plaies selon la revendication 10, dans lequel le dispositif d'accès au lit de la plaie (10) inclut un orifice à pression réduite (110) intégré à l'interface de pansement (20), de sorte que l'interface de pansement (20) définit deux chemins d'écoulement à travers elle, un premier associé au connecteur à valve auto-scellant (12) et un second associé à l'orifice à pression réduite (110) et dans lequel le système de soin des plaies (300) inclut une source de pression réduite (304) sous la forme d'une pompe à vide connectée à l'orifice à pression réduite (110) pour fournir une pression négative au niveau de la plaie.
